# FASCICULE DE BREVET EUROPEEN

(11) **EP 3 719 377 B1**
(45) Date de publication et mention de la délivrance du brevet: **09.11.2022**
(21) Numéro de dépôt: 20165798.8
(22) Date de dépôt: 26.03.2020
(51) Int. Cl.: F16L 37/407, F16L 37/413, F16L 37/42, F16L 3/12, A61M 39/26, F16L 37/60, A61M 39/10, A61M 16/08, A61M 39/24, F16B 5/02

(54) **ENSEMBLE MURAL COMPRENANT UNE PRISE DE DISTRIBUTION DE FLUIDE MÉDICAL À FIXATION ET D'INDEXAGE AMÉLIORÉS**
WANDANORDNUNG MIT EINEM ANSCHLUSS FÜR DIE AUSGABE EINES MEDIZINISCHEN FLUIDS MIT VERBESSERTER BEFESTIGUNG UND INDEXIERUNG
WALL ASSEMBLY COMPRISING A SOCKET FOR DISTRIBUTING MEDICAL FLUID WITH IMPROVED ATTACHMENT AND INDEXING

(30) Priorité: 04.04.2019 FR 1903612
(43) Date de publication de la demande: 07.10.2020
(73) Titulaire: Air Liquide Medical Systems, 92160 Antony (FR)
(72) Inventeur: CHEVEREAU, Christophe, 92160 ANTONY (FR); LOPEZ, Julien, 92160 ANTONY (FR); VADON, Gautier, 77173 Chevry-Cossigny (FR)
(74) Mandataire: Air Liquide

(56) Documents cités:
- EP-A1- 1 611 921
- EP-A2- 0 489 671
- WO-A1-2019/021434
- WO-A2-2008/128497
- KR-B1- 101 476 328

## Description

L'invention concerne un enemble de distribution de fluide mural, comprenant une prise murale de distribution de fluide médical à fixation et d'indexage/orientation améliorées, notamment une prise murale de distribution de gaz ou de vide, laquelle est destinée à être fixée à une paroi au sein d'un bâtiment hospitalier ou analogue pour fournir du gaz ou du vide (i.e. aspiration/dépression), l'ensemble comprenant aussi un boitier rigide protégeant la prise.

Il est usuel d'utiliser des prises de distribution de fluide pour distribuer les fluides, en particulier les gaz médicaux (i.e. un gaz pur ou un mélange gazeux) ou le vide (i.e. dépression < 1 atm), au sein des bâtiments hospitaliers. On les trouve notamment dans les chambres des patients, dans les salles d'opération ou de soins, ou d'autres pièces du bâtiment. Ces prises permettent de fournir les gaz médicaux véhiculés par les réseaux de canalisations de gaz parcourant les bâtiments hospitaliers, aux appareils et équipements utilisés pour traiter et soigner les patients au sein de ces bâtiments, en particulier les gaz thérapeutiques, tels l'oxygène, le protoxyde d'azote ou l'air, ou le vide médical (i.e. aspiration/dépression) permettant d'opérer des aspirations notamment de liquides biologiques, par exemple le sang ou d'autres liquides biologiques.

Les prises de distribution de fluide sont aussi couramment appelées « prises murales » ou « raccords muraux », car elles sont généralement montées soit directement sur les parois, c'est-à-dire les murs, cloisons ou analogues, des bâtiments hospitaliers, soit indirectement, par exemple en étant intégrées à un boitier ou analogue qui est lui-même monté sur une paroi. Les cas d'installations peuvent inclure aussi les "gaines tête de lit", utilisés au chevet du patient dans les chambres, et les "bras plafonniers", utilisés dans les blocs opératoires.

Le document EP 1 611 921 A1 propose une prise destinée à la mise en oeuvre de fluides médicaux, à raccorder à un système de distribution, sur laquelle un opérateur peut brancher ou débrancher un appareil destiné à alimenter en fluide médical spécifique.

Le document FR-A-2628820 propose une prise de distribution de fluide, appelée raccord à verrouillage automatique, comprenant un corps de prise de forme allongée comprenant un passage axial traversant axialement le corps de prise de sorte de relier fluidiquement une extrémité amont à une extrémité aval. Le fluide, tel un gaz médical ou le vide (i.e. dépression/aspiration) « circule » d'une extrémité à l'autre, lorsqu'un connecteur d'un appareil ou équipement médical, tel le connecteur d'une conduite de fluide, est raccordé mécaniquement et fluidiquement à la prise.

Le corps de prise comprend aussi une tubulure, c'est-à-dire un petit conduit, en communication fluidique avec le passage axial traversant le corps de prise et qui est par ailleurs solidarisée au corps de prise selon un axe perpendiculaire à l'axe du corps de prise. Cette tubulure vient se raccorder au réseau de gaz ou de vide de l'hôpital. Lorsque la prise murale est agencée sur une paroi, la tubulure doit être verticale, alors que le passage axial du corps de prise doit être horizontal.

Or, il a été constaté en pratique qu'un problème pouvait se poser lors du montage/fixation et surtout de l'indexage/orientation d'une telle prise murale sur une paroi au sein d'un bâtiment hospitalier. En effet, avec les prises actuelles, la fixation de la prise contraint l'orientation angulaire de la prise elle-même, c'est-à-dire son indexage, puisqu'il n'existe pas de degré de liberté entre l'orientation de la tubulure qui doit être verticale et le corps de prise. Cela conduit alors souvent à une prise mal positionnée, c'est-à-dire avec une tubulure non verticale.

Le problème est dès lors de proposer un ensemble amélioré de distribution de fluide mural, comprenant une prise de distribution de fluide, c'est-à-dire de gaz ou de vide, qui soit plus facile à fixer et à indexer/orienter de manière à ce que la tubulure faisant la jonction entre le réseau de gaz ou de vide de l'hôpital et le passage axial du corps de prise, soit verticale ou quasi-verticale.

La solution de l'invention porte sur un ensemble selon la revendication 1 de distribution de fluide mural. L'ensemble comprend une prise de distribution de fluide, à savoir de gaz ou de vide, comprenant :
- un corps de prise de forme allongée selon un axe (AA), comprenant un passage central traversant axialement (AA) le corps de prise, et une gorge annulaire aménagée dans la paroi périphérique du corps de prise et
- une tubulure, c'est-à-dire un petit conduit, en communication fluidique avec le passage central du corps de prise, la tubulure étant solidarisée au corps de prise selon un axe perpendiculairement à l'axe (AA) du corps de prise,
dans lequel la prise de distribution comprend en outre une pièce-étrier comprenant une ouverture bordée par un rebord arqué venant se loger dans la gorge annulaire du corps de prise, lorsque le corps de prise est positionné dans la pièce-étrier, la pièce-étrier comprenant en outre des perçages traversant la pièce-étrier, au moins l'un desdits perçages étant de forme circulaire et au moins l'un desdits perçages étant de forme oblongue.

Selon le mode de réalisation considéré, la prise de distribution de fluide peut comprendre l'une ou plusieurs des caractéristiques suivantes :
- les perçages de forme oblongue ont une forme (approximativement) ovale ou elliptique.
- le perçage de forme circulaire a un diamètre de l'ordre de 3 à 8 mm.
- les perçages de forme oblongue ont une longueur de l'ordre de 5 à 9 mm et une largeur de l'ordre de 3 à 8 mm.
- les moyens d'arrimage par vissage sont de préférence des vis, des boulons ou analogues.
- les perçages permettent de palier l'hyperstatisme de l'assemblage.
- la tubulure est rigide.
- le corps de prise est rigide.
- la tubulure est en métal ou alliage métallique.
- le corps de prise est en métal ou alliage métallique.
- le métal ou alliage métallique formant la tubulure et/ou le corps de prise est choisi parmi le cuivre, le laiton et l'acier.
- la tubulure forme tout ou partie de moyens de raccordement permettant de raccorder mécaniquement et fluidiquement une canalisation de fluide, en particulier une canalisation de fluide faisant partie d'un réseau de canalisations de fluide (i.e. gaz ou vide) agencé au sein d'un bâtiment hospitalier de sorte que ledit corps de prise soit en communication fluidique avec ladite canalisation de fluide.
- la tubulure comprend un lumen dans lequel circule le fluide, i.e. gaz ou dépression.
- la tubulure est fixée au corps de prise par soudage ou autre.
- le rebord arqué de la pièce-étrier a une forme complémentaire de la forme de la gorge annulaire du corps de prise.
- l'ouverture de la pièce-étrier a une section (sensiblement) circulaire, c'est-à-dire en cercle ou partie de cercle.
- la pièce-étrier est une plaque.
- la pièce-étrier est une plaque d'environ 0,5 mm à 5 mm d'épaisseur au travers de laquelle sont percés des perçages de fixation et une découpe arrondie constituant l'ouverture bordée par le rebord arqué, de préférence de 2 à 3 mm d'épaisseur.
- le boitier rigide agencé autour du corps de prise est un boitier de protection servant notamment à protéger la prise contre les poussières et les chocs éventuels.
- elle comporte un guide-embout agencé coaxialement dans le corps de prise.
- le guide-embout comprend un logement axial comprenant un clapet de tête mobile au sein dudit logement axial, un siège de clapet et un élément élastique.
- le guide-embout est monté extractible, c'est-à-dire démontable, dans le corps de prise.
- le clapet de tête est normalement repoussé contre le siège de clapet par l'élément élastique.
- le guide-embout extractible comprend une gorge annulaire aménagée dans sa paroi périphérique externe et un premier joint d'étanchéité annulaire agencé dans ladite gorge annulaire.
- le guide-embout est en un matériau rigide choisi parmi les alliages de cuivre, en particulier le laiton nickelé, et les alliages de zinc, d'aluminium et de magnésium, en particulier le zamak.
- le clapet de tête est mobile axialement (i.e. selon l'axe AA) au sein du logement axial, de préférence mobile en translation, en particulier coulissant.
- le clapet de tête, le siège de clapet et l'élément élastique forment tout ou partie d'éléments de contrôle du passage de gaz.
- l'élément élastique comprend un ressort ou analogue, par exemple un ressort métallique de forme cylindrique ou tronconique.
- l'élément élastique forme manchon autour d'au moins une partie du clapet de tête.
- un second joint d'étanchéité, tel un joint torique, est agencé autour du clapet de tête.
- le second joint d'étanchéité coopère avec le siège de clapet pour assurer une étanchéité fluidique entre clapet de tête et siège de clapet, lorsque le clapet de tête est totalement repoussé contre le siège de clapet par l'élément élastique.
- le premier et/ou le second joint d'étanchéité est en élastomère, en silicone ou autre.
- le guide-embout est fixé par vissage dans le corps de prise.
- le clapet de tête comprend un logement interne borgne comprenant un orifice axial et un ou plusieurs orifices latéraux en communication fluidique avec le logement interne borgne, de préférence au moins 3 ou 4 orifices latéraux.
- les orifices latéraux sont agencés en couronne autour du clapet de tête
- le guide-embout forme manchon autour du clapet de tête.
- le guide-embout est formé d'au moins deux sous-unités fixées l'une à l'autre.
- le guide-embout comprend un logement axial se terminant par un orifice proximal.
- l'orifice axial du clapet de tête et l'orifice proximal du guide-embout sont agencés coaxiaux.
- le clapet de tête a (au moins en partie) une forme générale tubulaire.
- le clapet de tête comprend une portion avant tubulaire au sein de laquelle est aménagée le logement interne borgne.
- les orifices latéraux sont percés au travers de la paroi latérale de la portion avant tubulaire.
- le passage central du corps de prise relie fluidiquement une extrémité distale à une extrémité proximale.
- le guide-embout est monté de manière extractible, c'est-à-dire qu'il peut être extrait ou démonté de la prise.
- l'élément élastique est agencé autour d'au moins une partie de la partie distale (ou partie arrière) du clapet de tête.
- l'élément élastique vient appuyer, en particulier par l'une de ses extrémités, sur un épaulement agencé autour du clapet de tête et solidaire de celui-ci, notamment un épaulement de forme annulaire.
- le guide-embout comprend un fond, de préférence un fond borgne, sur lequel vient appuyer, en particulier par l'autre de ses extrémités, l'élément élastique.
- un système de sécurité, agencé dans le passage central, comprend une bille-clapet ou analogue et une chambre à bille dans laquelle est logée la bille-clapet, et un siège de clapet à bille coopérant avec ladite bille-clapet pour contrôler le passage de fluide.
- le guide-embout comprend une expansion venant coopérer avec la bille-clapet du système de sécurité.

Avantageusement, l'ensemble de distribution de fluide mural est destiné à être monté sur une paroi, tel un mur ou une cloison, en particulier au sein d'un bâtiment hospitalier, comprenant une prise de distribution de fluide avec une pièce-étrier selon l'invention et un boitier rigide agencé autour du corps de prise de ladite prise de manière à la protéger.

Selon le mode de réalisation considéré, ensemble de distribution de fluide mural de l'invention peut comprendre l'une ou plusieurs des caractéristiques suivantes :
- le boitier rigide comprend une ouverture sur le dessus permettant de laisser passer la tubulure de la prise, en position verticale.
- le boitier rigide a une forme de préférence parallélépipédique rectangle.

Selon un autre aspect, l'invention porte aussi sur un bâtiment hospitalier comprenant une paroi, tel un mur ou une cloison, sur laquelle est monté un ensemble de distribution de fluide mural selon l'invention.

De préférence, l'ensemble de distribution de fluide mural selon l'invention est fixé à la paroi par des moyens à vis et de la pièce-étrier.

L'ensemble selon l'invention peut être fixé à une paroi verticale, tel un mur ou une cloison, ou encore dans une gaine tête de lit ou bras plafonnier, par un procédé de fixation dans lequel :
a) on fixe la pièce-étrier au moyen d'éléments de vis vissés de manière lâche à la paroi verticale au travers des perçages de la pièce-étrier,
b) on positionne la prise de distribution de fluide dans la pièce-étrier de sorte que le rebord arqué bordant l'ouverture de la pièce-étrier vienne se loger dans la gorge annulaire du corps de prise,
c) on indexe la prise de distribution de fluide par rapport à la pièce-étrier de manière à ce que la tubulure de la prise soit en position verticale, et
d) on opère un serrage complet des éléments de vis de manière à maintenir fermement et solidairement la prise, l'étrier et la paroi verticale, les uns aux autres.

On agence ensuite autour de la prise de distribution de fluide le boitier rigide qui comprend une ouverture sur le dessus permettant de laisser passer la tubulure en position verticale.

On positionne ensuite la face avant du boitier portant le couvercle pivotant et l'orifice central traversé par une partie de la prise de distribution de fluide et on fixe l'ensemble au moyen d'un écrou ou analogue venant se fixer au corps de prise (par vissage) en venant repousser la face avant de boitier contre le boitier rigide et à les maintenir en position assemblée.

L'invention va maintenant être mieux comprise grâce à la description détaillée suivante, faite à titre illustratif mais non limitatif, en référence aux figures annexées parmi lesquelles :
- [Fig. 1] représente une vue de face d'un mode de réalisation d'une prise de distribution de gaz ;
- [Fig. 2] représente une vue en coupe de côté de la prise de la [Fig. 1] ; et
- [Fig. 3] représente une vue de côté dissociée de la prise des [Fig. 1] et [Fig. 2].

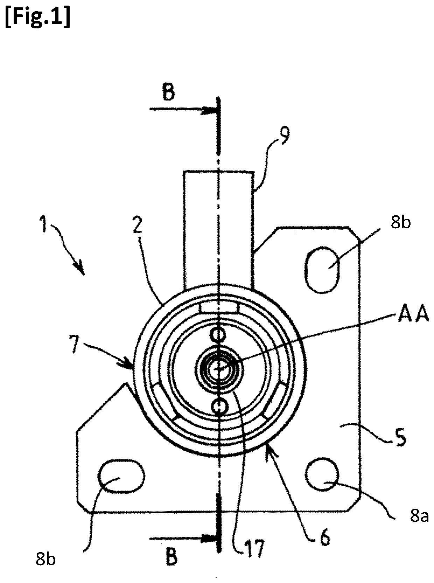

La [Fig. 1] représente une vue de face d'un mode de réalisation d'une prise de distribution 1 de gaz comprenant un corps de prise 2 de forme allongée, selon un axe longitudinal AA, à savoir un axe horizontal lorsque la prise 1 est fixée à une paroi verticale (i.e. mure, cloison...) d'un bâtiment hospitalier et ce, que ce soit directement ou via un élément-support intermédiaire, tel un caisson ou analogue, lui-même fixé à la paroi.

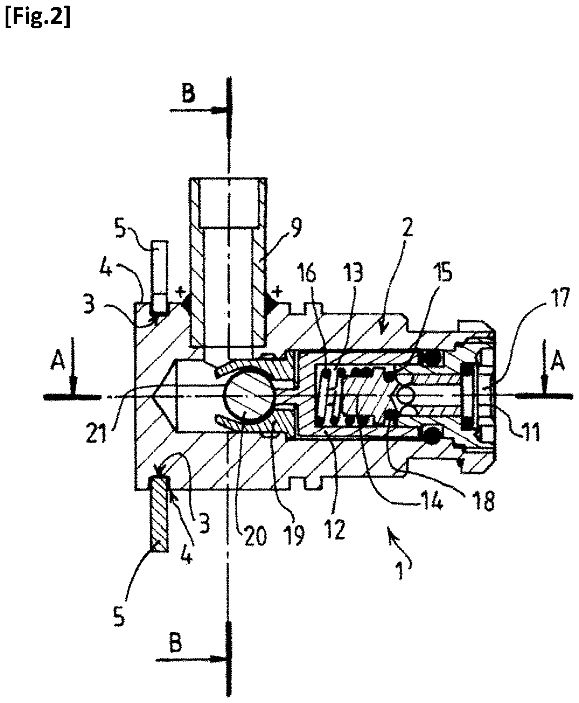

Comme visible sur la [Fig. 2], le corps de prise 2 est traversé axialement (selon axe AA) par un passage axial 11, i.e. passage central, formant un logement interne au sein duquel sont agencés, de manière extractible, des éléments de contrôle du passage de gaz. Une telle architecture est classique.

Les éléments de contrôle du passage de gaz comprennent un guide-embout 12 comprenant un logement axial 13, un clapet de tête 14 mobile selon l'axe AA, i.e. coulissant en translation, au sein dudit logement axial 13 et un orifice proximal 17 de passage de fluide, i.e. gaz ou vide, lequel orifice 17 est de section circulaire. Par exemple, lorsque le fluide est un gaz (i.e. gaz pur ou mélange gazeux), celui-ci est distribué, c'est-à-dire sort du corps 2 de la prise 1, par l'orifice proximal 17.

Est également prévu un élément élastique 16, tel un ressort cylindrique ou analogue, agencé dans le logement axial 13. De préférence, l'élément élastique 16 est agencé autour de la portion arrière du clapet de tête 14 et vient prendre appui, d'une part, sur le fond borgne du logement axial 13 et, d'autre part, sur un épaulement annulaire solidaire du clapet de tête 14, par exemple formé dans la paroi périphérique externe du clapet de tête 14. L'élément élastique 16 permet de normalement repousser le clapet de tête 14 contre un siège de clapet 15 aménagé dans le guide-embout 12, par exemple au sein de la paroi interne délimitant le logement axial 13 du guide-embout 12, de sorte de contrôler le passage de fluide dans le corps de prise 2.

Avantageusement, un joint torique 18 ou tout autre moyen d'étanchéité analogue est agencé autour du clapet de tête 14, lequel vient coopérer avec le siège de clapet 15 pour assurer une étanchéité fluidique entre le clapet de tête 14 et le siège de clapet 15 lorsqu'ils sont en contact l'un avec l'autre, c'est-à-dire lorsque le clapet de tête 14 est repoussé contre le siège de clapet 15 par l'élément élastique 16, par exemple un ressort cylindrique agencé autour du clapet de tête 14 mobile et agissant sur l'épaulement du clapet de tête 14.

Le clapet de tête 14 comprend, quant à lui, un logement interne borgne comprenant un orifice axial en communication fluidique avec le logement interne borgne, ainsi qu'un ou plusieurs orifices latéraux, par exemple 4 orifices latéraux, répartis en couronne autour de celui-ci, qui sont eux aussi en communication fluidique avec le logement interne borgne de sorte que le fluide (i.e. gaz ou vide) puisse entrer ou sortir dudit logement interne borgne du clapet de tête 14, via ces orifices latéraux.

Avantageusement, la prise 1 de distribution de fluide comprend aussi un système de sécurité 19-21 agencé dans le passage axial 11 comprenant une bille-clapet 20 ou analogue (i.e. demi-sphère ou sphère) et une chambre à bille 21 dans laquelle est logée la bille-clapet 20, et un siège 19 de clapet à bille coopérant avec ladite bille-clapet 20 pour contrôler le passage de fluide. Dans le cas d'une prise de vide, la bille-clapet 20 est préférentiellement une demi-sphère coopérant avec un ressort de rappel.

De préférence, le siège 19 de clapet est aménagé dans la paroi interne de la chambre à bille 21. Ce système de sécurité 19-21 permet d'empêcher que le gaz ne puisse s'échapper de la prise 1 (selon le mode de réalisation présenté) ou que l'aspiration par le vide (i.e. dépression) ne se fasse (selon un autre mode de réalisation non présenté), c'est-à-dire que de l'air ne puisse entrer dans le réseau de vide relié à la prise 1 et y faire remonter la pression (< 1 atm) qui y règne, lorsque les éléments de contrôle du passage de gaz, typiquement le guide-embout 12, sont démontés et extraits du corps de prise 2, notamment lors d'une opération de vérification, de maintenance ou de remplacement. La bille-clapet 20 fait office de clapet de sécurité venant, sous l'effet de la pression fluidique ou de la dépression (i.e. vide) s'exerçant sur la bille-clapet 20, appuyer sur et obturer le siège de clapet 19, lorsque les éléments de contrôle du passage de gaz, typiquement le guide-embout 12, sont extraits du corps 2 de prise, de sorte d'interrompre toute liaison fluidique.

A l'inverse, quand le guide-embout 12 est monté dans le corps de prise 2, il vient appuyer sur la bille-clapet 20 pour la décoller du siège de clapet 19 et autoriser ainsi la communication fluidique au travers de l'orifice ou canal de liaison. Ceci peut se faire via une tige axiale, formant une expansion ou analogue, faisant saillie sur la surface externe arrière du guide-embout 12 et solidaire de celui-ci, qui est orientée de sorte de traverser axialement l'orifice ou canal de liaison pour venir appuyer sur la bille-clapet 20 et ainsi la décoller du siège de clapet 19.

Il est à noter que la bille-clapet 20 peut avoir différentes formes, notamment une forme de sphère comme sur [FIG. 1], de demi-sphère ou une autre forme adaptée, par exemple ovale, ovoïde ou autre.

Par ailleurs, selon le mode de réalisation choisi, le guide-embout 12 peut être formé de plusieurs sous-unités fixées l'une à l'autre, par exemple par vissage, emboitement, soudage ou autre.

Par ailleurs, le guide-embout 12 peut être réalisé en métal ou alliage métallique, en particulier, en alliage de cuivre, tel le laiton, de préférence nickelé, i. e. laiton nickelé, ou en alliage de zinc, d'aluminium et de magnésium, en particulier le zamak.

Le raccordement fluidique de la prise 1 au réseau de canalisations de fluide se fait via une tubulure 9, c'est-à-dire un petit conduit de gaz, qui est en communication fluidique, via son lumen, avec, d'une part, ledit réseau et, d'autre part, avec le passage central interne 11 de la prise 1. La tubulure 9 est solidarisée au corps de prise 2 selon un axe (BB) perpendiculairement à l'axe (AA) du corps de prise 2, comme illustré en [Fig. 2], par exemple fixée par soudage.

La tubulure 9 et le corps de prise 2 sont rigides, de préférence en métal ou alliage métallique, par exemple en laiton, en cuivre ou en acier.

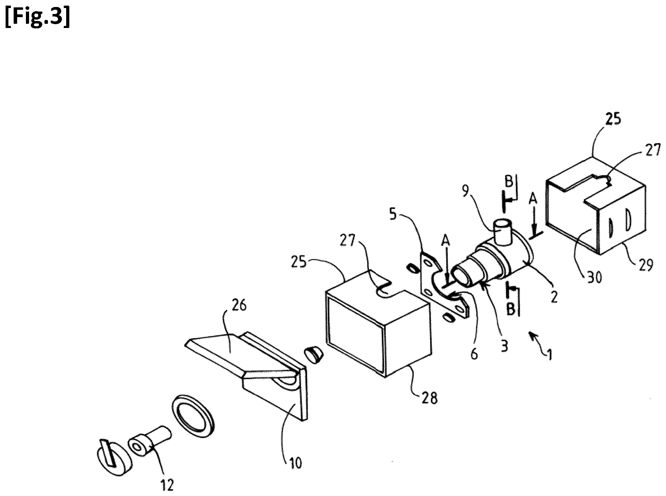

Ainsi, lorsque la prise murale 1 est fixée à une paroi, l'axe BB de la tubulure 9 est sensiblement vertical et l'axe (AA) du corps de prise 2 est sensiblement horizontal, comme montré en [Fig. 3].

Selon la présente invention, afin de faciliter la fixation et surtout l'indexage du corps 2 de la prise 1 de manière à ce que la tubulure 9 faisant la jonction entre le réseau de gaz ou de vide de l'hôpital et le passage axial 11 du corps 2 de prise, soit verticale ou quasi-verticale, il est prévu une gorge annulaire 3 aménagée dans la paroi périphérique 4 du corps de prise 2, et une pièce-étrier 5 comprenant une ouverture 7 bordée par un rebord arqué 6, i.e. une découpe arrondie, venant se loger dans la gorge annulaire 3 du corps de prise 2, lorsque le corps de prise 2 est positionné dans la pièce-étrier 5, comme illustré en [Fig. 1] à [Fig. 3].

La pièce-étrier 5 comprend en outre des moyens de fixation à une paroi, directement ou via un élément-support intermédiaire, tel un caisson mural ou analogue. Comme visible sur les [Fig. 1] et [Fig. 3], les moyens de fixation comprennent par exemple des perçages 8 destinés à recevoir des vis, des boulons ou tout autre élément ou moyen d'arrimage par vissage.

La pièce-étrier 5 comporte un perçage 8a de forme circulaire et deux perçages 8b, 8c de forme oblongue, par exemple sensiblement ovale ou ellipsoïdale.

La présence de 3 perçages permet de palier l'hyperstatisme du montage résultant du centrage de l'étrier sur le corps de prise et de la fixation de l'étrier au mur par les 3 vis.

De préférence, le perçage circulaire du mur est d'abord réalisé par l'opérateur, ce qui permet de fixer la pièce-étrier 5 à la paroi, i.e. au mur.

La géométrie particulière selon l'invention des perçages 8b et 8c de la pièce-étrier 5 permet une latitude dans la précision du perçage réalisée par le technicieninstallateur, de préférence de l'ordre de +/-1mm, par rapport à la longueur des trous oblongs. Ainsi, si la perceuse "rippe" légèrement durant l'opération de perçage, l'installation de la prise murale reste possible grâce à cet intervalle de tolérance.

Par ailleurs, la pièce-étrier 5 peut comprendre une plaque d'environ 0,5 mm à 5 mm d'épaisseur, de préférence de l'ordre de 2 à 3 mm, au travers de laquelle sont percés les perçages 8 de fixation et la découpe arrondie constituant l'ouverture 7 bordée par le rebord arqué 6.

Préférentiellement, le corps de prise 2 a une section circulaire, c'est-à-dire qu'il forme un cylindre dans la portion de corps portant la gorge annulaire 3, c'est-à-dire que la gorge annulaire 3 est aménagée sur toute la périphérie circulaire externe de la paroi périphérique 4 corps de prise 2.

Le rebord arqué 6 de la pièce-étrier 5 a avantageusement une forme, i.e. un profil, complémentaire de la forme de la gorge annulaire 3 du corps de prise 2 de sorte que le rebord arqué 6 vienne épouser les contours (i.e. vienne se centrer) de la gorge annulaire 3 du corps de prise 2 lorsque le rebord arqué 6 est positionné dans la gorge annulaire 3, de préférence le rebord arqué 6 a une forme de partie de cercle comme visible en [Fig. 3], par exemple de l'ordre de 20 à 30 mm de diamètre, préférentiellement de l'ordre de 25 à 27 mm. Le rebord arqué 6 est constitué d'une ouverture ou découpe arrondie en demi-cercle (i.e. angle de l'ordre de 180°) afin de permettre l'insertion et le maintien du corps de prise 2 via la gorge annulaire 3. Ce rebord est prolongé de part et d'autre de section rectilignes, ici de l'ordre 10 mm.

Grâce à un tel agencement, il est extrêmement aisé et rapide de non seulement fixer le corps de prise 2 à la pièce-étrier 5 mais aussi d'indexer ces deux parties l'une par rapport à l'autre de manière à ce que la tubulure 9 soit verticale. Ceci est opéré par un opérateur, lors du montage de la prise 1 sur la paroi, via une simple orientation selon un mouvement de rotation horaire ou antihoraire du corps 2 dans la pièce-étrier 5 jusqu'à mettre la tubulure 9 en position désirée, i.e. verticale.

Cette rotation horaire ou antihoraire engendre un déplacement relatif du corps 2 dans la pièce-étrier 5, c'est-à-dire du rebord 6 dans la gorge 4, donc permet un indexage correct du corps 2 dans la pièce-étrier 5.

Une fois le corps 2 correctement positionné et indexé dans la pièce-étrier 5, il suffit que l'opérateur agissent sur les moyens de fixation, notamment opère un serrage des vis, boulons ou autres éléments ou moyens d'arrimage par vissage passant au travers des perçages 8 traversant la pièce-étrier 5 et venant s'arrimer à la paroi pour solidariser fermement la prise 1 à la paroi.

Pour la protéger des poussières notamment, la prise murale 1 est agencée dans un boitier rigide 25 périphérique définissant un compartiment interne 30 pour recevoir la prise 1 et la protéger, comme illustré en [Fig. 3].

Le boitier rigide 25 est parallélépipédique. Il peut être formé de plusieurs parties se connectant les uns aux autres, par exemple deux sous-unités parallélépipédiques 28, 29 venant s'emboiter l'une dans l'autre, ainsi qu'une face avant 10 portant un couvercle pivotant 26 donnant accès au compartiment interne 30 et à la prise 1 qui s'y trouve. Toutefois, la sous-unité arrière 29 (qui est percée de trous de passage des vis ou analogue) formant la partie arrière de boitier est optionnelle et est omise dans certains cas, par exemple en cas d'intégration de l'ensemble dans un mur.

En fait, la sous-unité 28 formant le corps principal du boitier rigide 25 est en quelque sorte prise en « sandwich » entre la pièce-étrier 5 et la face avant 10 portant le couvercle pivotant 26. Cette dernière 10 est quant à elle maintenue par un écrou ou analogue. La pièce-étrier 5 vient toutefois se fixer directement à la paroi, c'est-à-dire à un mur par exemple, par vissage au travers des orifices 8.

Par ailleurs, le boitier rigide 25 comprend une ouverture 27 sur le dessus permettant de laisser passer la tubulure 9 qui est indexée en position verticale.

Un ensemble selon l'invention est conçu pour fournir soit des gaz thérapeutiques, tels l'oxygène, le protoxyde d'azote, l'air ou tout autre gaz ou mélange gazeux, soit du vide médical (i.e. dépression) servant à opérer des aspirations notamment de liquides biologiques, par exemple le sang ou d'autres liquides biologiques.

## Revendications

1. Ensemble de distribution de fluide mural (1 ,25), comprenant une prise de distribution de fluide (1) comprenant :
- un corps de prise (2) de forme allongée selon un axe (AA), comprenant un passage central (11) traversant axialement (AA) le corps de prise (2), et une gorge annulaire (3) aménagée dans la paroi périphérique (4) du corps de prise (2),
- une tubulure (9) en communication fluidique avec le passage central (11) du corps de prise (2), la tubulure (9) étant solidarisée au corps de prise (2) selon un axe (BB) perpendiculairement à l'axe (AA) du corps de prise (2), et
- une pièce-étrier (5) comprenant une ouverture (7) bordée par un rebord arqué (6) venant se loger dans la gorge annulaire (3) du corps de prise (2), lorsque le corps de prise (2) est positionné dans la pièce-étrier (5), la pièce-étrier (5) comprenant en outre des perçages (8a, 8b, 8c) traversant la pièce-étrier (5), lesdits perçages (8a, 8b, 8c) étant dimensionnés pour recevoir des moyens d'arrimage par vissage,
**caractérisé en ce qu'**il comprend en outre un boitier rigide (25) agencé autour du corps de prise (2), le boitier rigide ayant une forme générale parallélépipédique et comprenant :
- un compartiment interne (30) comprenant la prise de distribution de fluide (1), et une face avant (10) portant un couvercle pivotant (26) donnant accès audit compartiment interne (30), la face avant (10) comprenant un orifice central traversé par une partie de la prise de distribution de fluide (1), la face avant (10) étant fixée au corps de la prise (2) par un élément d'écrou venant se visser sur le corps de prise (2), et
- une ouverture (27) sur le dessus du boitier (25) permettant de laisser passer la tubulure (9) de la prise (1), en position verticale,
et dans lequel au moins l'un des perçages (8a, 8b, 8c) de la pièce-étrier (5) est de forme circulaire et au moins deux desdits perçages (8a, 8b, 8c) sont de forme oblongue.

2. Ensemble selon la revendication 1, **caractérisé en ce que** le rebord arqué (6) de la pièce-étrier (5) a une forme complémentaire de la forme de la gorge annulaire (3) du corps de prise (2).

3. Ensemble selon la revendication 1, **caractérisé en ce que** les perçages (8b, 8c) de forme oblongue ont une forme ovale ou elliptique.

4. Ensemble selon la revendication 1, **caractérisé en ce que** le perçage (8a) de forme circulaire a un diamètre de 3 à 8 mm.

5. Ensemble selon l'une des revendications 1 ou 3, **caractérisé en ce que** les perçages (8b, 8c) de forme oblongue ont une longueur de 5 à 9 mm et une largeur de 3 à 8 mm.

6. Ensemble selon la revendication 1, **caractérisée en ce que** l'ouverture (7) de la pièce-étrier (5) a une section circulaire.

7. Ensemble selon l'une des revendications 1 ou 6, **caractérisé en ce que** la pièce-étrier (5) est une plaque, de préférence en acier.

8. Ensemble selon la revendication 1, **caractérisé en ce que** les perçages (8a, 8b, 8c) sont dimensionnés pour recevoir des moyens d'arrimage par vissage choisis parmi les vis, les boulons ou analogues.

9. Ensemble selon l'une des revendications 1 ou 7, **caractérisé en ce que** la pièce-étrier (5) est une plaque de 0,5 mm à 5 mm d'épaisseur, de préférence entre 2 et 3 mm, au travers de laquelle sont percés des perçages (8) de fixation et une découpe arrondie constituant l'ouverture (7) bordée par le rebord arqué (6).

10. Bâtiment hospitalier comprenant une paroi, tel un mur ou une cloison, sur laquelle est monté un ensemble de distribution de fluide mural (1, 25) selon l'une des revendications précédentes.

## Patentansprüche

1. Wandanordnung zur Fluidabgabe (1, 25), welche einen Fluidabgabeanschluss (1) umfasst, welcher umfasst:
- ein Anschlusskörper (2) mit einer längs einer Achse (AA) lang gestreckten Form, welcher einen zentralen Durchgang (11), der den Anschlusskörper (2) axial (AA) durchquert, und eine Ringnut (3), die in der Umfangswand (4) des Anschlusskörpers (2) angeordnet ist, umfasst,
- einen Stutzen (9), der mit dem zentralen Durchlass (11) des Anschlusskörpers (2) in Fluidverbindung steht, wobei der Stutzen (9) mit dem Anschlusskörper (2) entlang einer Achse (BB) senkrecht zur Achse (AA) des Anschlusskörpers (2) fest verbunden ist, und
- ein Bügelstück (5), das eine Öffnung (7) umfasst, die von einer bogenförmigen Randleiste (6) umrandet wird, die in der Ringnut (3) des Anschlusskörpers (2) aufgenommen wird, wenn der Anschlusskörper (2) in dem Bügelstück (5) positioniert wird, wobei das Bügelstück (5) außerdem Bohrungen (8a, 8b, 8c) umfasst, die das Bügelstück (5) durchqueren, wobei die Bohrungen (8a, 8b, 8c) dafür bemessen sind, Mittel zur Befestigung durch Verschraubung aufzunehmen,
**dadurch gekennzeichnet, dass** sie außerdem ein starres Gehäuse (25) umfasst, das um den Anschlusskörper (2) herum angeordnet ist, wobei das starre Gehäuse allgemein quaderförmig ist und umfasst:
- einen Innenraum (30), der den Fluidabgabeanschluss (1) umfasst, und eine Vorderseite (10), die einen schwenkbaren Deckel (26) trägt, der den Zugang zu dem Innenraum (30) ermöglicht, wobei die Vorderseite (10) einen zentralen Durchlass umfasst, der von einem Teil des Fluidabgabeanschlusses (1) durchquert wird, wobei die Vorderseite (10) am Anschlusskörper (2) durch ein Mutterelement befestigt ist, das auf den Anschlusskörper (2) aufgeschraubt wird, und
- eine Öffnung (27) auf der Oberseite des Gehäuses (25), welche ermöglicht, den Stutzen (9) des Anschlusses (1) in einer vertikalen Position durchzulassen,
und wobei mindestens eine der Bohrungen (8a, 8b, 8c) des Bügelstücks (5) kreisförmig ist und mindestens zwei dieser Bohrungen (8a, 8b, 8c) von länglicher Form sind.

2. Anordnung nach Anspruch 1, **dadurch gekennzeichnet, dass** die bogenförmige Randleiste (6) des Bügelstücks (5) eine Form aufweist, die zur Form der Ringnut (3) des Anschlusskörpers (2) komplementär ist.

3. Anordnung nach Anspruch 1, **dadurch gekennzeichnet, dass** die Bohrungen (8b, 8c) von länglicher Form eine ovale oder elliptische Form aufweisen.

4. Anordnung nach Anspruch 1, **dadurch gekennzeichnet, dass** die kreisförmige Bohrung (8a) einen Durchmesser von 3 bis 8 mm aufweist.

5. Anordnung nach einem der Ansprüche 1 bis 3, **dadurch gekennzeichnet, dass** die Bohrungen (8b, 8c) von länglicher Form eine Länge von 5 bis 9 mm und eine Breite von 3 bis 8 mm aufweisen.

6. Anordnung nach Anspruch 1, **dadurch gekennzeichnet, dass** die Öffnung (7) des Bügelstücks (5) einen kreisförmigen Querschnitt aufweist.

7. Anordnung nach einem der Ansprüche 1 bis 6, **dadurch gekennzeichnet, dass** das Bügelstück (5) eine Platte ist, vorzugsweise aus Stahl.

8. Anordnung nach Anspruch 1, **dadurch gekennzeichnet, dass** die Bohrungen (8a, 8b, 8c) dafür bemessen sind, Mittel zur Befestigung durch Verschraubung aufzunehmen, die aus Schrauben, Bolzen oder dergleichen gewählt sind.

9. Anordnung nach einem der Ansprüche 1 bis 7, **dadurch gekennzeichnet, dass** das Bügelstück (5) eine Platte mit einer Dicke von 0,5 mm bis 5 mm, vorzugsweise zwischen 2 und 3 mm ist, durch die hindurch Bohrungen (8) zur Befestigung und ein gerundeter Ausschnitt, der die von der bogenförmigen Randleiste (6) umrandete Öffnung (7) bildet, gebohrt sind.

10. Krankenhausgebäude, welches eine Wand, wie etwa eine Mauer oder eine Zwischenwand, umfasst, an welcher eine Wandanordnung zur Fluidabgabe (1, 25) nach einem der vorhergehenden Ansprüche angebracht ist.

## Claims

1. Wall-mounted fluid distribution assembly (1, 25), comprising a fluid distribution outlet (1) comprising:
- an outlet body (2) of elongate shape along an axis (AA), comprising a central passage (11) passing axially (AA) through the outlet body (2), and an annular groove (3) formed in the peripheral wall (4) of the outlet body (2),
- a pipe (9) in fluidic communication with the central passage (11) of the outlet body (2), the pipe (9) being secured to the outlet body (2) along an axis (BB) perpendicular to the axis (AA) of the outlet body (2), and
- a yoke piece (5) comprising an opening (7) bordered by an arcuate edge (6) that comes to be housed in the annular groove (3) of the outlet body (2), when the outlet body (2) is positioned in the yoke piece (5), the yoke piece (5) also comprising drilled holes (8a, 8b, 8c) passing through the yoke piece (5), said drilled holes (8a, 8b, 8c) being dimensioned so as to receive means for anchoring by screwing,
**characterized in that** it also comprises a rigid casing (25) arranged around the outlet body (2), the rigid casing having a generally parallelepipedal shape and comprising:
- an internal compartment (30) comprising the fluid distribution outlet (1), and a front face (10) bearing a pivoting cover (26) giving access to said internal compartment (30), the front face (10) comprising a central orifice that is passed through by a part of the distribution fluid outlet (1), the front face (10) being fastened to the body of the outlet (2) by a nut element that comes to be screwed onto the outlet body (2), and
- an opening (27) on the top of the casing (25) making it possible to let the pipe (9) of the outlet (1) pass, in the vertical position,
and wherein at least one of the drilled holes (8a, 8b, 8c) in the yoke piece (5) is of circular shape and at least two of said drilled holes (8a, 8b, 8c) are of oblong shape.

2. Assembly according to Claim 1, **characterized in that** the arcuate edge (6) of the yoke piece (5) has a shape complementary to the shape of the annular groove (3) of the outlet body (2).

3. Assembly according to Claim 1, **characterized in that** the drilled holes (8b, 8c) of oblong shape have an oval or elliptical shape.

4. Assembly according to Claim 1, **characterized in that** the drilled hole (8a) of circular shape has a diameter of 3 to 8 mm.

5. Assembly according to either of Claims 1 and 3, **characterized in that** the drilled holes (8b, 8c) of oblong shape have a length of 5 to 9 mm and a width of 3 to 8 mm.

6. Assembly according to Claim 1, **characterized in that** the opening (7) of the yoke piece (5) has a circular section.

7. Assembly according to either of Claims 1 and 6, **characterized in that** the yoke piece (5) is a plate, preferably made of steel.

8. Assembly according to Claim 1, **characterized in that** the drilled holes (8a, 8b, 8c) are dimensioned so as to receive means for anchoring by screwing that are chosen from screws, bolts or the like.

9. Assembly according to either of Claims 1 and 7, **characterized in that** the yoke piece (5) is a plate with a thickness of 0.5 mm to 5 mm, preferably between 2 and 3 mm, through which drilled holes (8) for fastening and a rounded cut-out constituting the opening (7) bordered by the arcuate edge (6) are drilled.

10. Hospital building comprising a wall, such as a partition or a panel, on which is mounted a wall-mounted fluid distribution assembly (1, 25) according to one of the preceding claims.
